(19) Europäisches Patentamt
European Patent Office
Office européen des brevets

(11) Veröffentlichungsnummer: **0 086 403**
**A2**

(12) # EUROPÄISCHE PATENTANMELDUNG

(21) Anmeldenummer: **83101006.1**

(22) Anmeldetag: **03.02.83**

(51) Int. Cl.³: **C 07 D 263/24**, A 61 K 31/42, C 07 C 93/06

(30) Priorität: **16.02.82 DE 3205457**

(43) Veröffentlichungstag der Anmeldung: **24.08.83 Patentblatt 83/34**

(84) Benannte Vertragsstaaten: **AT BE CH DE FR GB IT LI NL SE**

(71) Anmelder: **Merck Patent Gesellschaft mit beschränkter Haftung, Frankfurter Strasse 250, D-6100 Darmstadt (DE)**

(72) Erfinder: **Kirchlechner, Richard, Dr., Im Sennfeld 19, D-8200 Rosenheim (DE)**
Erfinder: **Jonas, Rochus, Dr., Telemannweg 24, D-6100 Darmstadt (DE)**

(54) **Oxazolidin-2-one.**

(57) Die Erfindung betrifft neue Oxazolidin-2-one der allgemeinen Formel I,

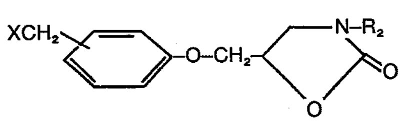

I

worin
X Cl, Br, J oder $OR^1$,
$R^1$ H, Alkenyl, Alkinyl, Alkoxyalkyl oder Alkenyloxyalkyl mit jeweils 2–6 C-Atomen oder Cycloalkyl mit 3–8 C-Atomen und
$R^2$ Alkyl oder Hydroxyalkyl mit jeweils 1–6 C-Atomen, Cycloalkyl mit 3–8 C-Atomen, unsubstituiertes Aralkyl oder im Arylrest ein- bis dreifach durch Alkyl, Alkoxy, OH und/oder Cl oder einfach durch Methylendioxy substituiertes Aralkyl mit jeweils insgesamt 7–15 C-Atomen bedeuten.

Sie eignen sich zur Bekämpfung von Herzkrankheiten und als Zwischenprodukte.

EP 0 086 403 A2

0086403

# Oxazolidin-2-one

Die Erfindung betrifft neue Oxazolidin-2-one der allgemeinen Formel I

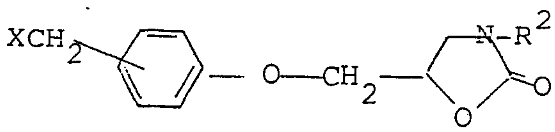
I

worin

X Cl, Br, J oder $OR^1$

$R^1$ H, Alkenyl, Alkinyl, Alkoxyalkyl oder Alkenyloxyalkyl mit jeweils 2-6 C-Atomen oder Cycloalkyl mit 3-8 C-Atomen und

$R^2$ Alkyl oder Hydroxyalkyl mit jeweils 1-6 C-Atomen, Cycloalkyl mit 3-8 C-Atomen, unsubstituiertes Aralkyl oder im Arylrest ein- bis dreifach durch Alkyl, Alkoxy, OH und/oder Cl oder einfach durch Methylendioxy substituiertes Aralkyl mit jeweils insgesamt 7-15 C-Atomen

bedeuten.

Der Erfindung lag die Aufgabe zugrunde, neue Verbindungen aufzufinden, insbesondere solche, die zur Herstellung von Arzneimitteln verwendet werden können. Diese Aufgabe wurde durch die Bereitstellung der Verbindungen der Formel I gelöst.

Es wurde gefunden, daß diese Verbindungen bei guter Verträglichkeit wertvolle pharmakologische Eigenschaften besitzen. Vor allem zeigen sie isoprenalin-antagonistische Wirkungen auf die Herzfrequenz und den Blutdruck, z.B. von Meerschweinchen, Katzen oder Hunden, feststellbar z.B. nach der Methodik, die in der DT-AS 14 93 564 näher beschrieben ist. Einige der Verbindungen zeigen eine kardioselektive Wirkung. Ferner zeigen die Produkte Wirkungen auf das Zentralnervensystem sowie thrombozytenaggregationshemmende, antiarrhythmische und lipolysehemmende Wirkungen, die ebenfalls nach hierfür geläufigen Methoden ermittelt werden können.

Die Verbindungen können daher als Arzneimittelwirkstoffe in der Human- und Veterinärmedizin eingesetzt werden, insbesondere zur Prophylaxe und zur Behandlung von Herz-, Kreislauf- und Gefäßerkrankungen.

Gegenstand der Erfindung sind die neuen Verbindungen der Formel I.

In dieser Formel steht die Gruppe $XCH_2$- vorzugsweise in der p- oder in der o-Stellung des Benzolrings; sie kann jedoch auch in der m-Stellung stehen.

Der Rest X ist vorzugsweise Cl oder $OR^1$.

Falls der Rest $R^1$ Alkenyl bedeutet, so ist dieses vorzugsweise geradkettig und steht insbesondere für Allyl, ferner z.B. für Vinyl, Propenyl, Isopropyl, Butenyl (z.B. 1-Buten-1-yl, 1-Buten-2-yl, 2-Buten-1-yl, 2-Buten-2-yl, 3-Buten-1-yl, 3-Buten-2-yl), Isobutenyl (z.B. 2-Methyl-2-propen-1-yl), Pentenyl (z.B. 2-Penten-1-yl) oder Hexenyl (z.B. 2-Hexen-1-yl). Alkinyl ist vorzugsweise geradkettig und steht insbesondere für Propargyl, ferner für Ethinyl, 1-Propin-1-yl, Butinyl (z.B. 2-Butin-1-yl), Pentinyl (z.B. 2-Pentin-1-yl) oder Hexinyl (z.B. 2-Hexin-1-yl). Alkoxyalkyl bedeutet vorzugsweise Alkoxyethyl (worin die Alkoxygruppe 1 - 4 C-Atome hat), insbesondere 2-Methoxyethyl, 2-Ethoxyethyl, 2-Propoxyethyl oder 2-Isopropoxyethyl, ferner z.B. Alkoxypropyl, z.B. 2- oder 3-Methoxypropyl; Alkoxybutyl, z.B. 2-, 3- oder 4-Methoxybutyl; Alkoxypentyl, z.B. 5-Methoxypentyl. Alkenyloxyalkyl steht vorzugsweise für 2-Alkenyloxyethyl, insbesondere 2-Allyloxyethyl, ferner 2-Vinyloxyethyl oder 2-Propenyloxyethyl, ferner z.B. für Alkenyloxypropyl wie 2- oder 3-Allyloxypropyl. Cycloalkyl ist vorzugsweise Cyclopentyl oder Cyclohexyl, ferner z.B. Cyclopropyl, Cyclobutyl, 1-, 2- oder 3-Methylcyclopentyl, 1-, 2-, 3- oder 4-Methylcyclohexyl, Cycloheptyl oder Cyclooctyl.

Falls der Rest $R^2$ Alkyl bedeutet, so steht dieses vorzugsweise für verzweigtkettiges Alkyl insbesondere mit 3 oder 4 C-Atomen wie Isopropyl, Isobutyl oder tert.-Butyl, ferner z.B. für Methyl, Ethyl, n-Propyl, n-Butyl, sek.-Butyl, Pentyl wie 1-, 2- oder 3-Pentyl, Isopentyl, Neopentyl, tert.-Pentyl, Hexyl wie 1-, 2- oder 3-Hexyl oder Isohexyl. Hydroxyalkyl kann z.B. bedeuten: Hydroxymethyl, 1- oder 2-Hydroxyethyl, 1-, 2- oder 3-Hydroxypropyl, 1-Hydroxy-1-methylethyl, 1-Methyl-2-hydroxyethyl, 1-, 2-, 3- oder 4-Hydroxybutyl, 5-Hydroxypentyl

oder 6-Hydroxyhexyl. Falls $R^2$ Cycloalkyl bedeutet, so steht Cycloalkyl vorzugsweise für einen der oben angegebenen Reste. Aralkyl hat 7-15, vorzugsweise 7-11-Atome und ist vorzugsweise 2-Phenylethyl, 1,1-Dimethyl-2-phenylethyl, 2-Methyl-2-phenylethyl, 1-, 2- oder 3-Phenylpropyl, 1-Methyl-2-phenylpropyl, 1-, 2-, 3- oder 4-Phenylbutyl, 1- oder 2-Naphthylmethyl, 2-(1-Naphthyl)-ethyl oder 2-(2-Naphthyl)-ethyl.

Falls $R^2$ eine Aralkylgruppe bedeutet, so kann der Arylrest darin ein- bis dreifach durch Alkyl, Alkoxy, OH, F und/oder Cl oder einfach durch Methylendioxy substituiert sein. Falls Alkyl- oder Alkoxygruppen als Substituenten im Arylrest stehen, so haben diese 1 - 8, vorzugsweise 1 - 4 C-Atome; der substituierte Aralkylrest darf aber insgesamt nicht mehr als 15 C-Atome enthalten. Vorzugsweise hat der substituierte Aralkylrest insgesamt 7 - 11 C-Atome. Als Alkylgruppen kommen insbesondere Methyl, Ethyl, n-Propyl, Isopropyl, n-Butyl, Isobutyl, sek.- Butyl, tert.-Butyl, n-Pentyl, Isopentyl, n-Hexyl oder n-Octyl in Frage, als Alkoxygruppen insbesondere Methoxy, ferner Ethoxy, n-Propoxy, Isopropoxy, n-Butoxy, Isobutoxy, sek.-Butoxy, n-Propoxy, Isopropoxy, n-Butoxy, Isobutoxy, sek.-Butoxy, tert.-Butoxy, n-Pentyloxy, Isopentyloxy, n-Hexyloxy, n-Heptyloxy oder n-Octyloxy. Bevorzugte substituierte Arylreste sind vor allem Alkoxyphenyl wie o-, m- oder p-Methoxyphenyl, o-, m- oder p-Ethoxyphenyl, Dialkoxyphenyl wie 2,3-, 2,4-, 2,5-, 2,6-, 3,5- oder besonders bevorzugt 3,4-Dimethoxyphenyl, Trialkoxyphenyl wie 3,4,5-Trimethoxyphenyl, Methylendioxyphenyl wie 3,4-Methylendioxyphenyl, ferner z.B. Alkylphenyl wie o-, m- oder p-Tolyl, o-, m- oder p-Ethylphenyl, o-, m- oder p-Isopropylphenyl, o-, m- oder p-tert.-Butylphenyl,

Dialkylphenyl wie 2,3-, 2,4-, 2,5-, 2,6-, 3,4- oder 3,5-Dimethylphenyl, Trialkylphenyl wie 2,4,6-Trimethylphenyl, 2,6-Dimethyl-4-tert.-butylphenyl, o-, m- oder p-Hydroxyphenyl, Dihydroxyphenyl wie 3,4-Dihydroxyphenyl, o-, m- oder p-Fluorphenyl, o-, m- oder p-Chlorphenyl. Die Arylreste können auch zwei verschiedene Substituenten tragen; Beispiele dafür sind 3-Methoxy-4-hydroxyphenyl und 3-Hydroxy-4-methoxyphenyl. Im einzelnen ist 2-(3,4-Dimethoxyphenyl)-ethyl ein besonders bevorzugter substituierter Aralkylrest. Weitere besonders bevorzugte substituierte Aralkylreste sind z.B. 2-(p-Methoxyphenyl)-ethyl, 2-(3,4,5-Trimethoxyphenyl)-ethyl und 2-(3,4-Methylendioxyphenyl)-ethyl.

Dementsprechend sind Gegenstand der Erfindung insbesondere diejenigen Verbindungen der Formel I, in denen mindestens einer der Reste X, $R^1$ und $R^2$ eine der vorstehend angegebenen bevorzugten Bedeutungen hat.

Gegenstand der Erfindung ist ferner ein Verfahren zur Herstellung von Verbindungen der Formel I nach Anspruch 1, dadurch gekennzeichnet, daß man eine Verbindung der allgemeinen Formel II

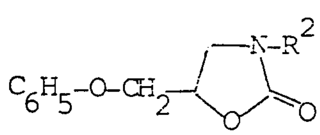

II

einer Halogenmethylierung unterzieht

oder daß man eine Verbindung der Formel III

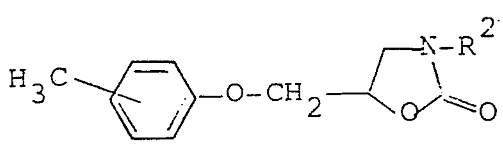

III

halogeniert und daß man gegebenenfalls eine erhaltene Verbindung der Formel I (X = Cl, Br oder J) durch Reaktion mit einer Verbindung der Formel $R^1$-OH oder einem ihrer reaktionsfähigen Derivate in eine Verbindung der Formel I (X = $OR^1$) überführt.

Die Herstellung der Verbindungen der Formel I erfolgt im übrigen nach an sich bekannten Methoden, wie sie in der Literatur (z.B. in den Standard-Werken wie Houben-Weyl, Methoden der Organischen Chemie, Georg-Thieme-Verlag, Stuttgart; Organic Reactions, John Wiley und Sons, Inc., New York) beschrieben sind, und zwar unter Bedingungen, wie sie für die genannten Umsetzungen bekannt und geeignet sind. Dabei kann man auch von an sich bekannten, hier nicht näher erwähnten Varianten Gebrauch machen.

Die Ausgangsstoffe der Formeln II und III sind teilweise bekannt, teilweise neu. Die neuen Ausgangsstoffe können nach an sich bekannten Verfahren, in der Regel in Analogie zu den bekannten Ausgangstoffen, hergestellt werden, beispielsweise durch Umsetzung entsprechender Aminoalkohole der Formel $Ar-O-CH_2CHOHCH_2-NHR^2$ (worin Ar Phenyl oder Tolyl bedeutet) mit einem Kohlensäurederivat, z.B. Diethylcarbonat.

Die Ausgangsstoffe können, falls erwünscht, auch in situ gebildet werden, derart, daß man sie aus dem Reaktionsgemisch nicht isoliert, sondern sofort weiter zu den Verbindungen der Formel I umsetzt.

Vor- und nachstehend haben die Reste X, $R^1$ und $R^2$ die bei Formel I angegebenen Bedeutungen, sofern nicht ausdrücklich etwas anderes angegeben ist.

Die Verbindungen der Formel I (X = Cl, Br oder J) sind vorzugsweise durch Halogenmethylierung, insbesondere Chlormethylierung, von II oder durch Halogenierung von III erhältlich.

Eine Halogenmethylierung von II gelingt durch Einwirkung von Formaldehyd, Paraformaldehyd oder Methylal in Gegenwart der entsprechenden Halogenwasserstoffsäure. Der Zusatz eines Katalysators wie $AlCl_3$, $ZnCl_2$, $H_2SO_4$ oder Essigsäure ist möglich, aber nicht unbedingt erforderlich. Zweckmäßig ist dagegen die Anwesenheit eines inerten Lösungsmittels, beispielsweise eines Kohlenwasserstoffs wie Petrolether, Hexan, Benzol oder Toluol, oder einer Carbonsäure wie Essigsäure. Man arbeitet im allgemeinen bei Temperaturen zwischen etwa -20 und etwa 120°, vorzugsweise zwischen 0 und 25°.

Eine Halogenierung von III gelingt beispielsweise mit einem Sulfurylhalogenid wie $SO_2Cl_2$ oder $SO_2Br_2$ bei Temperaturen zwischen etwa 100 und 150°, wobei Zusätze wie $PCl_3$ und/oder Peroxide von Vorteil sein können. Eine andere Methode besteht in der Umsetzung von III mit überschüssigem $BrCCl_3$ unter Bestrahlung.

Eine erhaltene Verbindung der Formel I (X = Cl, Br, oder J) kann durch Hydrolyse, zweckmäßig in Gegenwart einer Base wie NaOH oder KOH und eines inerten Lösungsmittels wie Methanol, Ethanol oder Isopropanol bei Temperaturen zwischen etwa 0 und etwa 30°, in eine Verbindung der Formel I (X = OH) umgewandelt werden.

Durch Veretherung mit Alkoholen der Formel $R^1$-OH (worin $R^1$ von H verschieden ist) sind aus den genannten Verbindungen der Formel I (X = Cl, Br, J oder OH) die übrigen Verbindungen der Formel I erhältlich. Diese

Veretherung gelingt in Anwesenheit oder Abwesenheit eines zusätzlichen inerten Lösungsmittels bei Temperaturen zwischen etwa 0 und etwa 200, vorzugsweise zwischen 10 und 50°. Als inerte Lösungsmittel eignen sich diejenigen, die für derartige Veretherungen aus der Literatur bekannt sind, beispielsweise Ether wie Diethylether, Diisopropylether, Tetrahydrofuran oder Dioxan; Kohlenwasserstoffe wie Benzol, Toluol oder Xylol; halogenierte Kohlenwasserstoffe wie Dichlormethan oder Chloroform; Ketone wie Aceton; Amide wie Dimethylformamid; Sulfoxide wie Dimethylsulfoxid; besonders zweckmäßig ist es jedoch, einen Überschuß des Alkohols der Formel $R^1$-OH als Lösungsmittel zu verwenden. Es können auch Gemische dieser Lösungsmittel benutzt werden. Die Alkohole der Formel $R^1$-OH werden zweckmäßig in Form der entsprechenden Natrium- oder Kaliumalkoholate eingesetzt. Verwendet man die freien Alkohole, so kann sich auch der Zusatz eines sauren Katalysators empfehlen, z.B. einer anorganischen Säure (wie Schwefelsäure, Polyphosphorsäure, Bromwasserstoffsäure, Chlorwasserstoffsäure) und/oder einer organischen Säure (wie Ameisensäure oder p-Toluolsulfonsäure).

Die Verbindungen der Formel I können ein oder mehrere Asymmetriezentren besitzen. Sie können daher bei ihrer Herstellung als Racemate oder, falls optisch aktive Ausgangsstoffe verwendet werden, auch in optisch aktiver Form erhalten werden. Weisen die Verbindungen zwei oder mehr Asymmetriezentren auf, dann fallen sie bei der Synthese im allgemeinen als Gemische von Racematen an, aus denen man die einzelnen Racemate, beispielsweise durch Umkristallisieren aus inerten Lösungsmitteln, in reiner Form isolieren kann. Erhaltene Racemate können, falls erwünscht, nach an sich bekannten Methoden mechanisch oder chemisch in ihre optischen Antipoden getrennt

werden. Vorzugsweise werden aus dem Racemat durch Umsetzung mit einem optisch aktiven Trennmittel Diastereomere gebildet. Die verschiedenen Formen der Diastereomeren können in an sich bekannter Weise, z.B. durch fraktionierte Kristallisation, getrennt, und die optisch aktiven Verbindungen der Formel I können in an sich bekannter Weise aus den Diastereomeren in Freiheit gesetzt werden.

Gegenstand der Erfindung ist ferner die Verwendung von Verbindungen der Formel I als Zwischenprodukt bei der Synthese von Phenoxyamino-propanolen der Formel IV

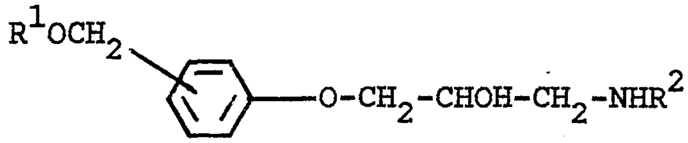

IV

in die sie durch Hydrolyse, vorzugsweise mit wässerigalkoholischen Basen wie NaOH oder KOH bei Temperaturen zwischen etwa 0 und etwa 50$^{o}$, leicht umgewandelt werden können.

Verbindungen der Formel IV (worin $R^1$ von H verschieden ist) sind aus der DE-OS 26 45 710 bekannt. Diese Substanzen sind wertvolle ß-Rezeptorenblocker, insbesondere die Verbindung IV ($R^1$ = 2-Isopropoxyethyl, $R^2$ = Isopropyl; "Bisoprolol"). Sie werden besonders vorteilhaft auf folgendem Wege hergestellt: 1-Phenoxy-2,3-epoxypropan wird mit Aminen der Formel $R^2$-$NH_2$ zu Aminoalkoholen der Formel $C_6H_5$-O-$CH_2$CHOH-$CH_2$-$NHR^2$ umgesetzt, die mit Diethylcarbonat in Oxazolidin-2-one der Formel II übergeführt werden. Chlormethylierung liefert I (X = Cl); daraus entsteht durch Veretherung I (X = $OR^1$, wobei $R^1$ von H verschieden ist). Hydrolyse liefert schließlich IV. Dieser Syntheseweg zeichnet sich durch hohe Ausbeuten, niedrige Kosten und die Möglichkeit aus, daß alle Zwischenprodukte und das Endprodukt IV besonders rein erhalten werden können.

Gegenstand der Erfindung ist ferner die Verwendung der Verbindungen der Formel I zur Herstellung pharmazeutischer Zubereitungen, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere Pharmazeutische Zubereitungen, enthaltend mindestens eine Verbindung der Formel I.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Glycerintriacetat, Gelatine, Kohlenhydrate wie Lactose oder Stärke, Magnesiumstearat, Talk, Vaseline. Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder

Aromastoffe enthalten. Sie können, falls erwünscht, auch einen oder mehrere weitere Wirkstoffe enthalten, z.B. ein oder mehrere Vitamine.

Gegenstand der Erfindung ist ferner die Verwendung der Verbindungen der Formel I bei der Bekämpfung von Krankheiten, insbesondere von Herzkrankheiten, sowie ihre Verwendung bei der therapeutischen Behandlung des menschlichen oder tierischen Körpers.

Dabei werden die erfindungsgemäßen Substanzen in der Regel in Analogie zu bekannten, im Handel befindlichen Herz- und Kreislaufmitteln, insbesondere ß-Rezeptorenblockern, z.B. Metoprolol oder Bisoprolol, verabreicht, vorzugsweise in Dosierungen zwischen etwa 0,5 und 200 mg, insbespndere zwischen 2 und 50 mg pro Dosierungseinheit. Die tägliche Dosierung liegt vorzugsweise zwischen etwa 0,01 und 4 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind die Temperaturen in °C angegeben.

Beispiel 1

In ein Gemisch von 48 g Paraformaldehyd und 1,2 l konzentrierter Salzsäure leitet man unter Kühlung Chlorwasserstoff bis zur Lösung ein. Anschließend wird eine Lösung

von 235 g 3-Isopropyl-5-phenoxymethyl-oxazolidin-2-on (F. 81 - 83°) in 750 ml Toluol zugegeben. Darauf leitet man bei 8 - 10° unter Rühren 5 Std. lang Chlorwasserstoff ein, trennt ab, wäscht die Toluolschicht mit gesättigter NaCl-Lösung, versetzt mit Petrolether und saugt das erhaltene 5-p-Chlormethyl-phenoxymethyl-3-isopropyl-oxazolidin-2-on ab; F. 89 - 92° (aus Toluol).

Beispiel 2

Analog Beispiel 1 erhält man mit 48 %iger Bromwasserstoffsäure an Stelle von Salzsäure und mit Bromwasserstoff an Stelle von Chlorwasserstoff das 5-p-Brommethyl-phenoxymethyl-3-isopropyl-oxazolidin-2-on.

Beispiel 3

Ein Gemisch von 249 g 3-Isopropyl-5-p-tolyloxymethyl-oxazolidin-2-on (erhältlich aus 1-Isopropylamino-3-p-tolyloxy-propan-2-ol und Diethylcarbonat), 5 g $PCl_3$ und 0,5 g Benzamid wird unter Rühren bei 135° innerhalb 6 Stunden mit 3 g Benzoylperoxid und 155 g $SO_2Cl_2$ versetzt. Man kühlt ab, arbeitet mit Ethylacetat/gesättigter NaCl-Lösung auf und erhält 5-p-Chlormethyl-phenoxymethyl-3-isopropyl-oxazolidin-2-on, F. 89 - 92°.

Analog erhält man mit $SO_2Br_2$ an Stelle von $SO_2Cl_2$ das 5-p-Brommethyl-phenoxymethyl-3-isopropyl-oxazolidin-2-on.

Beispiel 4

Man läßt 1 g 5-p-Chlormethyl-phenoxymethyl-3-isopropyl-oxazolidin-2-on über Nacht bei 20° mit einer Lösung von 0,8 g NaOH in 10 ml Ethanol und 10 ml Wasser stehen,

entfernt das Ethanol unter vermindertem Druck und filtriert das ausgefallene 5-p-Hydroxymethyl-phenoxymethyl-3-isopropyl-oxazolidin-2-on ab.

Beispiel 5

Unter Rühren und $N_2$ löst man 2,53 g Na in 75 ml 2-Isopropoxy-ethanol und versetzt dann mit einer Lösung von 28,4 g 5-p-Chlormethyl-phenoxymethyl-3-isopropyl-oxazolidin-2-on in 115 ml 2-Isopropoxy-ethanol. Man rührt 90 Minuten, gibt Essigsäure bis pH 7 hinzu, dampft ein und nimmt den Rückstand in $CH_2Cl_2$ auf. Man wäscht mit Wasser, trocknet über $Na_2SO_4$, dampft ein und erhält 5-(p-2-Isopropoxyethoxymethyl-phenoxymethyl)-3-isopropyl-oxazolidin-2-on; F. 77 - 78° (aus Toluol/Petrolether).

Beispiel 6

Man arbeitet wie in Beispiel 5 beschrieben, setzt jedoch 0,5 g KJ hinzu. Intermediär entsteht 5-p-Jodmethyl-phenoxymethyl-3-isopropyl-oxazolidin-2-on. Als Endprodukt erhält man ebenfalls 5-(p-2-Isopropoxyethoxymethyl-phenoxymethyl)-3-isopropyl-oxazolidin-2-on, F. 77 - 78°.

Beispiele 7 bis 14

Analog Beispiel 5 erhält man durch Veretherung mit den entsprechenden Alkoholen:

7. 5-(p-Allyloxymethyl-phenoxymethyl)-3-[2-(3,4-dimethoxyphenyl)-ethyl]-oxazolidin-2-on.

8. 3-(2-Phenylethyl)-5-(o-propargyloxymethyl-phenoxymethyl)-oxazolidin-2-on.

9. 5-(o-2-Allyloxyethoxymethyl-phenoxymethyl)-3-(1,1-dimethyl-2-phenylethyl)-oxazolidin-2-on.

10. 5-(o-Allyloxymethyl-phenoxymethyl)-3-isopropyl-oxazolidin-2-on.

11. 5-(m-Allyloxymethyl-phenoxymethyl)-3-isopropyl-oxazolidin-2-on.

12. 3-tert.-Butyl-5-(o-2-methoxyethoxymethyl-phenoxymethyl-oxazolidin-2-on.

13. 3-tert.-Butyl-5-(p-2-isopropoxyethoxymethyl-phenoxymethyl-oxazolidin-2-on.

14. 5-(o-Cyclopentoxymethyl-phenoxymethyl)-3-isopropyl-oxazolidin-2-on.

Verwendungsbeispiel:

Man kocht 1 g 5-(p-2-Isopropoxyethoxymethyl-phenoxymethyl)-3-isopropyl-oxazolidin-2-on mit 10 ml 17 %iger ethanolischer KOH 90 Minuten unter $N_2$, dampft ein, arbeitet mit wässeriger NaCl-Lösung/Ethylacetat auf und erhält öliges 1-(p-2-Isopropoxyethoxymethylphenoxy-methyl)-3-isopropylamino-propan-2-ol; Fumarat, F. 100 - 101° (aus Ethylacetat).

Die nachstehenden Beispiele betreffen pharmazeutische Zubereitungen, die Verbindungen der Formel I enthalten:

Beispiel A: Tabletten

Ein Gemisch von 1 kg 5-(p-2-Isopropoxyethoxymethyl-phenoxymethyl)-3-isopropyl-oxazolidin-2-on, 40 kg

Lactose, 12 kg Kartoffelstärke, 2 kg Talk und 1 kg Magnesiumstearat wird in üblicher Weise zu Tabletten gepreßt, derart, daß jede Tablette 5 mg Wirkstoff enthält.

Beispiel B: Dragees

Analog Beispiel A werden Tabletten gepreßt, die anschließend in üblicher Weise mit einem Überzug aus Saccharose, Kartoffelstärke, Talk, Tragant und Farbstoff überzogen werden.

Beispiel C: Kapseln

Man füllt 10 kg 5-(p-2-Isopropoxymethyl-phenoxymethyl)-3-isopropyl-oxazolidin-2-on in üblicher Weise in Hartgelatinekapseln, so daß jede Kapsel 20 mg Wirkstoff enthält.

Analog sind Tabletten, Dragees oder Kapseln erhältlich, die einen oder mehrere der übrigen Wirkstoffe der Formel I enthalten.

Patentansprüche:

1. Oxazolidin-2-one der allgemeinen Formel I

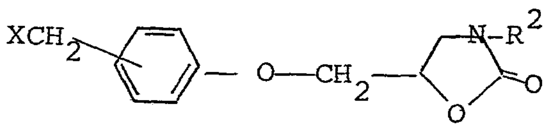

I

worin

X Cl, Br, J oder $OR^1$,

$R^1$ H, Alkenyl, Alkinyl, Alkoxyalkyl oder Alkenyloxyalkyl mit jeweils 2-6 C-Atomen oder Cycloalkyl mit 3-8 C-Atomen und

$R^2$ Alkyl oder Hydroxyalkyl mit jeweils 1-6 C-Atomen, Cycloalkyl mit 3-8 C-Atomen, unsubstituiertes Aralkyl oder im Arylrest ein- bis dreifach durch Alkyl, Alkoxy, OH und/oder Cl oder einfach durch Methylendioxy substituiertes Aralkyl mit jeweils insgesamt 7-15 C-Atomen

bedeuten.

2. 5-(p-2-Isopropoxyethoxymethyl-phenoxymethyl)-3-isopropyl-oxazolidin-2-on.

3. Verfahren zur Herstellung von Verbindungen der allgemeinen Formel I nach Anspruch 1, dadurch gekennzeichnet, daß man eine Verbindung der allgemeinen Formel II

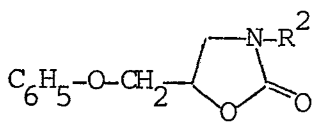

II

worin

$R^2$ die bei Formel I angegebene Bedeutung hat, einer Halogenmethylierung unterzieht oder daß man

eine Verbindung der allgemeinen Formel III

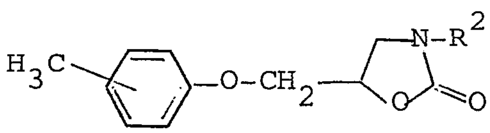

III

worin $R^2$ die bei Formel I angegebene Bedeutung hat halogeniert und daß man gegebenenfalls eine erhaltene Verbindung der Formel I (X = Cl, Br oder J) durch Reaktion mit einer Verbindung der allgemeinen Formel $R^1$-OH (worin $R^1$ die bei Formel I angegebene Bedeutung hat) oder einem ihrer reaktionsfähigen Derivate in eine Verbindung der Formel I (X = $OR^1$) überführt.

4. Verwendung von Verbindungen der Formel I nach Anspruch 1 als Zwischenprodukte bei der Synthese von Phenoxyamino-propanolen der Formel IV

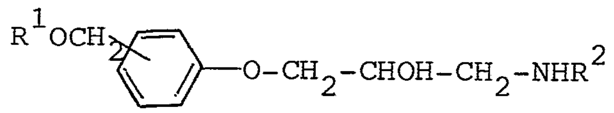

IV

worin

$R^1$ und $R^2$ die bei Formel I angegebene Bedeutung haben.

5. Verfahren zur Herstellung pharmazeutischer Zubereitungen, dadurch gekennzeichnet, daß man eine Verbindung der Formel I zusammen mit mindestens einem festen, flüssigen und/oder halbflüssigen Träger- oder Hilfsstoff und gegebenenfalls in Kombination mit einem oder mehreren weiteren Wirkstoff(en) in eine geeignete Dosierungsform bringt.

6. Pharmazeutische Zubereitung, gekennzeichnet durch einen Gehalt an mindestens einer Verbindung der Formel I.

7. Verbindungen der allgemeinen Formel I nach Anspruch 1 zur Bekämpfung von Krankheiten.

8. Verwendung von Verbindungen der Formel I bei der Bekämpfung von Herzkrankheiten.